# FASCICULE DE BREVET EUROPEEN

(11) **EP 0 486 403 B1**
(45) Date de publication et mention de la délivrance du brevet: **15.06.1994**
(21) Numéro de dépôt: 91420348.4
(22) Date de dépôt: 03.10.1991
(51) Int. Cl.: A61F 2/34

(54) **Cotyle pour prothèse de hanche**
Prothese für eine Hüftgelenkspfanne
Prosthetic hip cup

(30) Priorité: 13.11.1990 FR 9014400
(43) Date de publication de la demande: 20.05.1992
(73) Titulaire: IMPACT, F-01800 Charnoz (FR)
(72) Inventeur: Augoyard, Marc, F-69005 Lyon (FR); Bascoulergue, Gérard, F-62520 Le Touquet (FR); Basso, Maurice, F-43000 Le Puy (FR); Bertocchi, René, F-69006 Lyon (FR); Courcelles,, F-69570 Dardilly (FR); Debiesse, Jean-Louis, F-38200 Vienne (FR); Eyraud, Guy, F-38150 Peage du Roussillon (FR); Fayard, Jean Philippe, F-42170 St Just-St Rambert (FR); Melere, Gilles, F-74000 Annecy (FR); Millon, Joseph, F-74490 La Ravoire (FR); Passot, Jean Paul, F-42530 St Genest Lerpt (FR); Peyrot, Jacques, F-69005 Lyon (FR); Pintore, Ernesto, I-84100 Salerno (IT); Relave, Marc, F-42580 l'Etrat (FR); Roussouly, Pierre, F-69005 Lyon (FR); Collomb, Jean, F-26000 Porte les Valence (FR); Majou, Claude, F-01800 Meximieux (FR)
(74) Mandataire: Laurent, Michel

(56) Documents cités:
- EP-A- 0 169 978
- EP-A- 0 353 171
- GB-A- 1 415 736
- US-A- 4 676 798
- US-A- 4 695 282

## Description

L'invention concerne un nouveau type de cotyle perfectionné pour prothèse de hanche.

Comme on le sait, une prothèse de hanche comprend essentiellement deux parties, à savoir respectivement une tige destinée à être insérée dans le fémur à remplacer ou à renforcer, portant à son extrémité libre une tête sphérique, dans laquelle vient s'articuler un cotyle, lui-même engagé dans l'aile illiaque.

Le plus généralement, le cotyle est réalisé en deux parties distinctes, à savoir respectivement :
- une cupule dite de "soutien", destinée à être insérée dans l'aile illiaque ;
- et un noyau dit "de frottement", destiné à s'insérer étroitement dans la cupule de maintien, et dont la face interne concave reçoit la tête sphérique de la tige de prothèse.

En pratique, la cupule de soutien est rigide et est réalisée notamment en métal, tel qu'en un alliage de titane, alors que le noyau de frottement est le plus généralement réalisé en matière plastique, telle que notamment en polyéthylène.

Dans le document FR-A-2 634 644, on a décrit une cupule de soutien dont la face convexe externe présente d'une part, des têtons expansibles et d'autre part, une pluralité de fentes d'expansion de longueur croissante, ouvertes sur l'équateur, disposées sur des demi grands cercles passant par le pôle.

Bien qu'utilisé avec succès, ce type de cotyle présente toutefois certains inconvénients. En effet, l'architecture de l'ensemble reste anisotrope, ce qui gêne l'opération de vissage du noyau de frottement et nécessite un outil de pose spécial. Comme par ailleurs, les chevilles expansibles reposent sur la cupule par l'intermédiaire de bossages, il s'ensuit à l'usage la formation de pics de contrainte.

L'invention pallie ces inconvénients. Elle vise un cotyle perfectionné du type en question, qui soit plus facile à réaliser et à mettre en place.

Ce cotyle de prothèse de hanche, du type comprenant:
. une cupule de soutien, destinée à être insérée dans l'aile illiaque à renforcer (ou à remplacer), dont la face externe présente une pluralité de fentes d'expansion disposées sur des génératrices et qui sont ouvertes sur l'équateur, et dont la base interne est filetée ;
. un noyau de frottement, en forme générale de coupole, destiné du côté externe à être inséré dans la cupule de soutien, et du côté interne hémisphérique, à recevoir la tête fixe à l'extrémité du col prothétique de la tige de prothèse,

se caractérise :
- en ce que les fentes d'expansion sont symétriques, régulièrement espacées et débouchent sur le même parallèle ;
- en ce que l'ensemble comporte une bague intermédiaire filetée destinée à être vissée sur la base de la cupule de soutien pour l'expanser, et dont la face interne présente des bossages radiaux ;
- et en ce que le noyau de frottement présente des encoches disposées sur des génératrices régulièrement espacées, et une gorge annulaire sécante desdites encoches, l'ensemble encoches et gorge étant destiné à coopérer avec les bossages radiaux pour assurer le positionnement angulaire du noyau de frottement dans la cupule de maintien.

En d'autres termes, l'invention concerne un cotyle de prothèse de hanche formée d'une cupule de soutien, solidarisée à un noyau de frottement par une bague intermédiaire filetée à la base de la cupule de soutien, et dont la face interne présente des bossages qui coopèrent avec des encoches ménagées sur la face externe du noyau de frottement, dans laquelle est également ménagée une gorge, pour permettre le positionnement de ce noyau de frottement dans la cupule de soutien.

Avantageusement, en pratique :
- le noyau de frottement en forme de coupole comprend essentiellement trois parties accolées les unes aux autres, à savoir :
   . une première partie sensiblement hémisphérique, destinée à s'appuyer sur le fond de la cupule de soutien,
   . une portion cylindrique dans laquelle sont taillées les encoches et la gorge,
   . une collerette sur laquelle repose la bague intermédiaire d'expansion, et qui dépasse de la cupule de soutien, ladite collerette formant embase et étant biseautée par rapport à l'équateur de la cupule de soutien ;
- la face interne du noyau de frottement présente dans l'ordre :
   . une première portion hémisphérique destinée à recevoir la tête sphérique du col prothétique, raccordée à une portion cylindrique destinée à éviter la luxation de cette tête,
   . un chanfrein incliné de manière inverse à l'inclinaison de la collerette, destiné à autoriser l'inclinaison du col prothétique lors des rotations internes de la hanche ;
- la cupule de soutien présente des orifices ménagés entre deux fentes d'expansion contigües, destinés à recevoir des chevilles expansibles ou des vis à spongieux ;
- la cupule de soutien est en alliage de titane, alors que le noyau de frottement est en polyéthylène haute densité.

La manière dont l'invention peut être réalisée et les avantages qui en découlent ressortiront mieux de l'exemple de réalisation qui suit, à l'appui des figures annexées.

La figure 1 est une vue en perspective montrant les trois éléments caractéristiques séparés de l'invention.

La figure 2 est une vue de dessus de la cupule de soutien montrée en figure 3 dans sa variante à vis expansible.

La figure 4 est une vue en coupe de cette cupule de soutien.

Les figures 5 et 6 sont une représentation détaillée, respectivement en vue de dessus (figure 5) et en coupe (figure 6) de la bague intermédiaire d'expansion.

La figure 7 est une vue extérieure du noyau de frottement montré en vue de dessus (figure 8) et en coupe (figure 9).

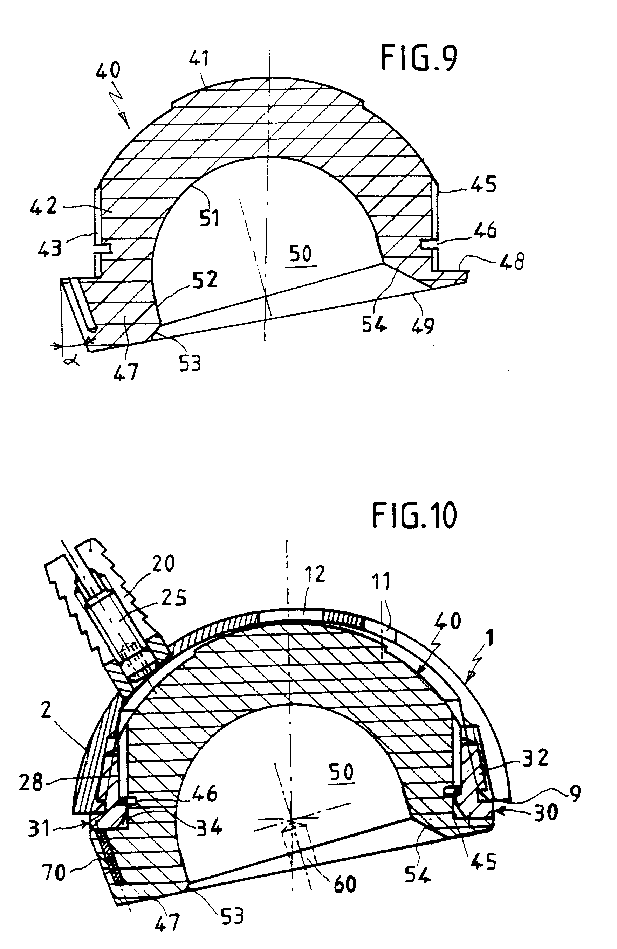

Enfin, la figure 10 est une représentation vue en coupe d'un cotyle de prothèse réalisée par l'assemblage de ces trois pièces caractéristiques.

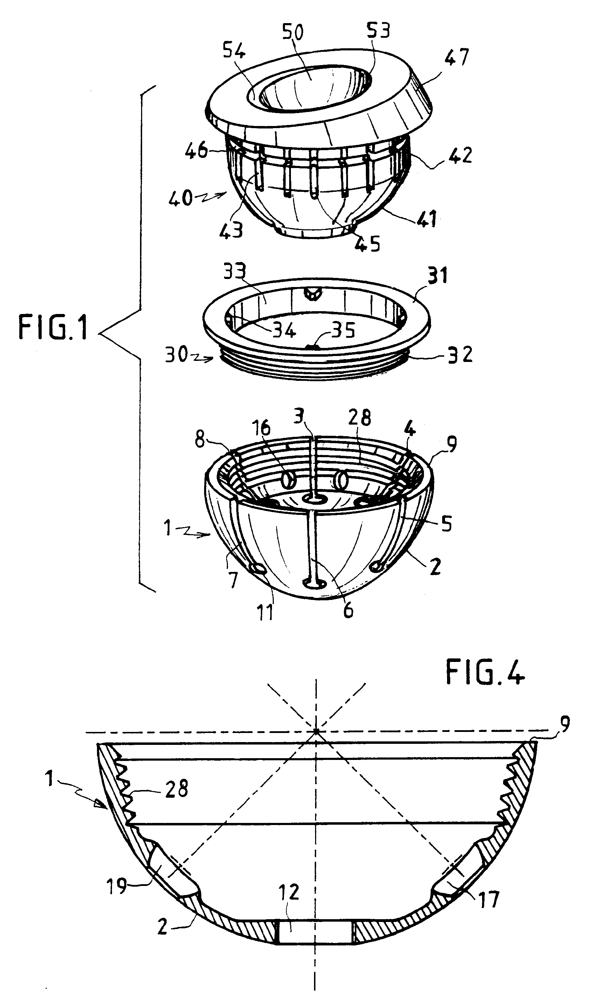

La cupule de soutien conforme à l'invention, désignée par la référence générale (1), montrée en détail aux figures 1, 2, 3 et 4, est réalisée en métal, notamment en un alliage de titane. Cette cupule présente une face externe (2) convexe, hémisphérique, dans laquelle sont ménagées six fentes d'expansion respectivement (3 à 8), régulièrement espacées, ouvertes sur l'équateur (9) et disposées sur des génératrices. Ces fentes d'expansion (3-8) sont symétriques et débouchent sur le même parallèle (10) disposé à environ 60° de latitude. Chaque fente (3-8) débouche dans un arrondi (11). Le pôle présente une ouverture (12). Les fentes (3-8), les arrondis (11) et le pôle (12) sont avantageusement réalisés par électroérosion.

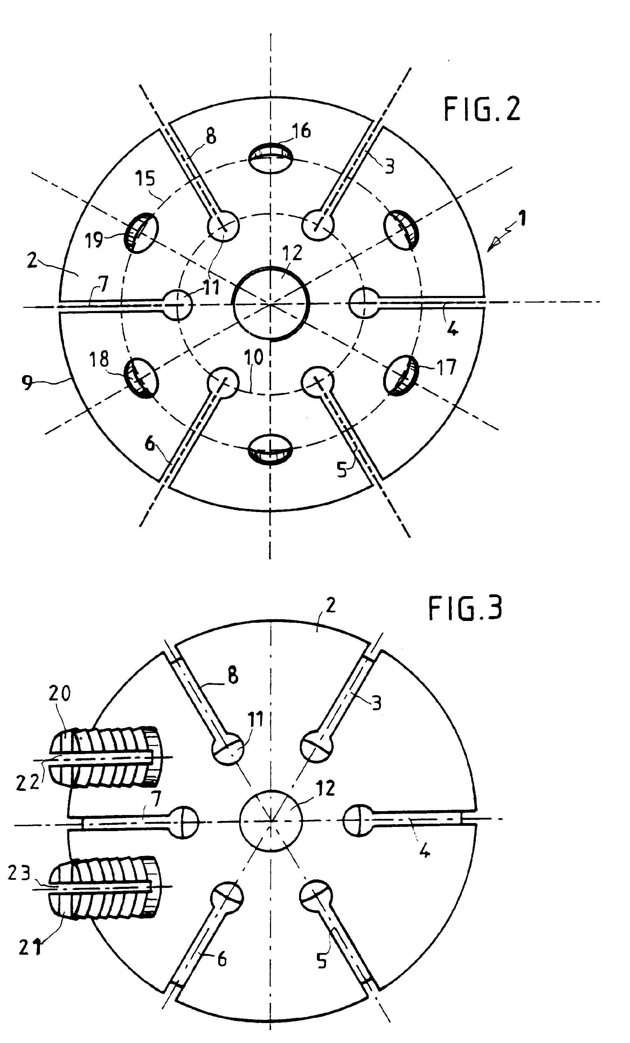

Chaque secteur, ménagé entre deux fentes (3-8) contigües présente, sur un parallèle (15), sensiblement disposé à environ 45° de latitude, des orifices traversants (16,17,18,19) destinés à recevoir soit des chevilles expansibles (20,21), comme montré aux figures 3 et 10, fendues longitudinalement (22,23), expansibles sous l'action de vis (25) actionnées depuis l'intérieur, soit des vis à spongieux à la base interne (28) de la cupule (1) et filetées.

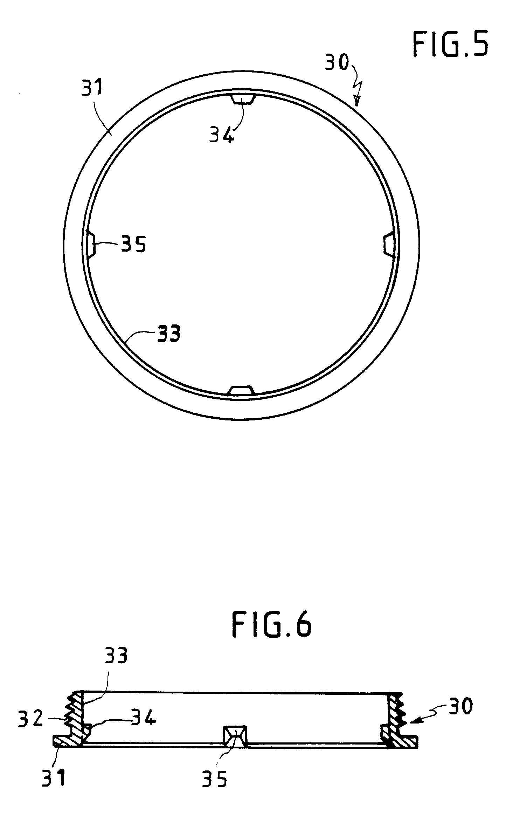

Selon une caractéristique de l'invention, l'ensemble comprend une bague intermédiaire désignée par la référence générale (30) montrée en détail aux figures 5 et 6. Cette bague intermédiaire d'expansion est avantageusement réalisée en métal, notamment en alliage de titane. Cette bague intermédiaire (30) présente une base (31) d'appui, raccordée à un filetage (32), destiné à venir s'engager dans le filetage (28) ménagé à la base interne de la cupule de soutien, de manière à expanser celle-ci et plus précisément, à écarter les fentes d'expansion (3-8). La face interne (33) de cette bague (30) cylindrique, présente à sa base, des bossages radiaux (34,35) en forme de saillie, par exemple décalés de 90°.

Ces bossages sont destinés à retenir le noyau de frottement par l'intermédiaire d'un clips métallique de verrouillage.

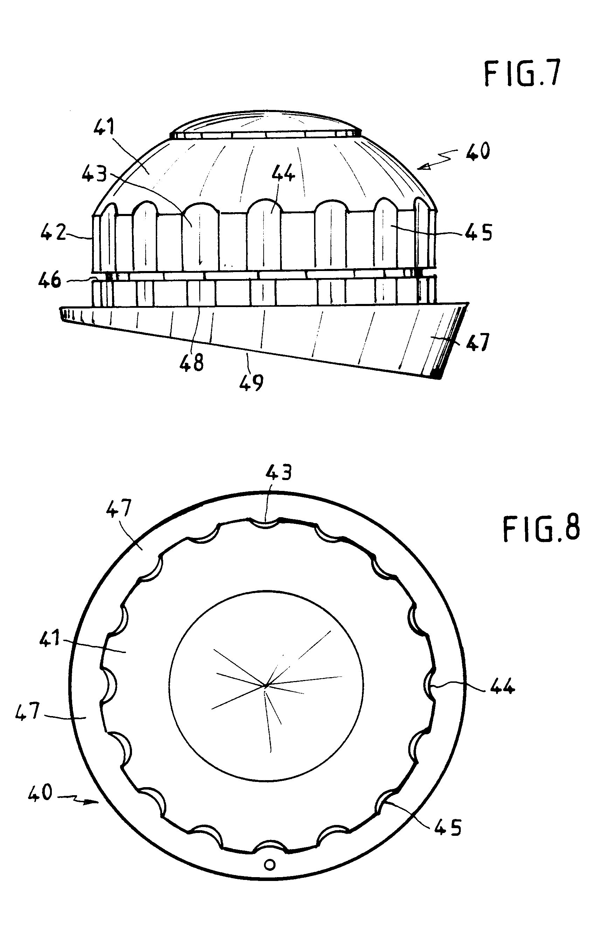

Le noyau de frottement caractéristique de l'invention, désigné par la référence générale (40), est montré en détail aux figures 1, 7, 8, 9 et 10. Ce noyau de frottement (40) en forme générale de coupole, est réalisé en une pièce monobloc en matière plastique, notamment en polyéthylène haute densité. Il comprend essentiellement trois parties accolées, obtenues par usinage, à savoir dans l'ordre, de haut en bas, une première portion sensiblement hémisphérique (41), destinée à venir s'emboiter sur le fond de la face interne de la cupule de soutien (1). Cette portion (41) est raccordée à une portion sensiblement cylindrique (42) dans laquelle sont taillées des encoches verticales (43,44,45), régulièrement espacées, disposées sur des génératrices, et destinées à coopérer avec les bossages radiaux (34,35) de la bague intermédiaire (30). Une gorge annulaire (46) coupe ces encoches (43,44,45), et est destinée à recevoir un clips métallique de verrouillage, qui prendra appui sur les encoches radiales (34,35) de la bague intermédiaire (30). Les cotes des encoches femelles (43,44,45) correspondent sensiblement aux dimensions des bossages en saillie mâles (34,35). La portion cylindrique (42) est raccordée à son tour, mais directement venue de moulage, à une collerette (47) formant embase, sur laquelle reposera la base (31) de la bague d'expansion intermédiaire (30). Cette collerette (47) (voir figure 10) dépasse la base (9) équatoriale de la cupule de soutien (1), et est biseautée dans la portion jouxtant cette embase. Plus exactement, l'angle formé entre les deux directions principales (48,49) de la collerette (47), est voisin de 10°, alors que le débord figuré par l'angle alpha sur la figure 9 est voisin de 20°.

Selon une autre caractéristique de l'invention, la face interne (50) de ce noyau de frottement présente dans l'ordre une première portion (51), hémisphérique, destinée à recevoir la tête sphérique du col prothétique, raccordée à une portion cylindrique (52), destinée à éviter la luxation de cette tête sphérique, laquelle est raccordée à son tour à un chanfrein incliné (53,54) de manière inverse à l'inclinaison de la collerette, destiné à autoriser l'inclinaison du col prothétique désigné par la référence générale (60) à la figure 10 lors des rotations internes de la hanche.

La référence (70) désigne un canal ménagé dans la collerette (47) destinée à recevoir un insert métallique repérable à la radiographie.

La mise en place du cotyle conforme à l'invention s'effectue de la manière suivante. De manière connue, le chirurgien effectue un fraisage sphérique dans le cotyle, dans lequel il positionne la cupule de soutien (1). Par passage à travers deux orifices contigus (18,19), il perce deux trous destinés à recevoir les chevilles expansibles (20,21). Il place les chevilles expansibles (20,21) sur la face externe (2) de la cupule de soutien (1), les met en place dans l'orifice et expanse ces chevilles par les vis appropriées (25). La cupule de soutien (1) se trouve ainsi positionnée.

Le chirurgien visse ensuite à la base (28) la bague intermédiaire (30), de manière à écarter les fentes d'expansion (3-8). Il introduit ensuite un noyau de frottement fantôme, identique à l'intérieur de la cupule de soutien, de manière à obtenir le réglage angulaire souhaité. Une fois cette position déterminée, le chirurgien retire le noyau de frottement fantôme et la remplace par un noyau de frottement (40) approprié, qui est clipsé sur les encoches radiales (46) lors de la mise en place des bossages radiaux (34,35) dans les encoches correspondantes (43,44,45) appropriées (voir figure 10).

Le cotyle de prothèse de hanche selon l'invention est facile à construire, facile à mettre en place et autorise des déplacements qu'il n'était pas possible d'obtenir jusqu'alors.

## Revendications

1. Cotyle pour prothèse de hanche, du type comprenant :
. une cupule de soutien (1), dont la face externe (2) présente une pluralité de fentes d'expansion (3-8) disposées sur des génératrices et qui sont ouvertes sur l'équateur (9), et dont la base interne (28) est filetée ;
. un noyau de frottement (40), en forme générale de coupole, destiné du côté externe (41) à être insérée dans la cupule de soutien (1), et du côté interne hémisphérique (50), à recevoir la tête fixe à l'extrémité du col prothétique de la tige de prothèse,
caractérisé :
- en ce que les fentes d'expansion (3-8) sont symétriques, régulièrement espacées et débouchent sur le même parallèle (10) ;
- en ce que l'ensemble comporte une bague intermédiaire (30) filetée, destinée à être vissée (32) sur la base (28) de la cupule de soutien pour expanser les fentes (3-8), et dont la face interne présente des bossages radiaux (34,35) ;
- et en ce que le noyau de frottement (40) présente des encoches (43-45) disposées sur des génératrices, régulièrement espacées, et une gorge annulaire (46) sécante desdites encoches (43-45), l'ensemble encoches (43-45) et gorge annulaire (46) étant destiné à coopérer avec les bossages radiaux (34,35) pour assurer le positionnement angulaire du noyau de frottement (40) dans la cupule de maintien (1).

2. Cotyle de prothèse de hanche selon la revendication 1, caractérisé en ce que le noyau de frottement (40) en forme générale de coupole, est monobloc et comprend essentiellement trois parties accolées, respectivement :
. une première partie (41) sensiblement hémisphérique, destinée à s'appuyer sur le fond de la cupule de soutien (1),
. une portion cylindrique (42), dans laquelle sont taillées les encoches (43-45) et la gorge (46),
. une collerette (47) sur laquelle repose la bague intermédiaire (30) d'expansion, et qui dépasse de la cupule de soutien, ladite collerette (47) formant embase et étant biseautée par rapport à l'équateur (9) de la cupule de soutien (1).

3. Cotyle de prothèse de hanche selon l'une des revendications 1 et 2, caractérisé en ce que la face interne (50) du noyau de frottement (40), présente dans l'ordre :
. une première portion hémisphérique (51), destinée à recevoir la tête sphérique du col prothétique,
. raccordée à une portion cylindrique (52) destinée à éviter la luxation de cette tête,
. raccordée à son tour à un chanfrein incliné (53,54) de manière inverse à l'inclinaison de la collerette (47), destiné à autoriser l'inclinaison du col prothétique (60) lors des rotations internes de la hanche.

4. Cotyle de prothèse de hanche selon l'une des revendications 1 à 3, caractérisé en ce que la cupule de soutien (1) présente des orifices (16-19) ménagés entre deux fentes d'expansion (3-8) contigues, destinées à recevoir des chevilles expansibles (20,21) ou des vis à spongieux.

5. Cotyle de prothèse de hanche selon l'une des revendications 1 à 4, caractérisé en ce que la cupule de soutien (1) et la bague intermédiaire d'expansion (30) sont en alliage de titane, alors que le noyau de frottement (40) est en polyéthylène haute densité.

## Patentansprüche

1. Gelenkpfanne für eine Hüftprothese, die folgendes aufweist:
. eine Stützkuppenschale (1), deren Außenseite (2) eine Vielzahl an Dehnschlitzen (3-8) aufweist, die längs Mantellinien angeordnet sind, und die zum Äquator (9) hin offen sind, wobei die innenliegende Basis (28) der Stützkuppenschale (1) mit einem Gewinde versehen ist;
. eine Reibnuß (40) in der allgemeinen Form einer Kuppel, die dazu vorgesehen ist, mit der Außenseite (41) in die Stützkuppenschale (1) eingeführt zu werden, und die dazu vorgesehen ist, in deren inneren halbkugelförmigen Seite (50) den an ein äußeres Ende des Prothesenhalses des Prothesenschaftes angebrachten Kopf aufzunehmen,
dadurch gekennzeichnet,
- daß die Dehnschlitze (3-8) symmetrisch, sowie gleichmäßig voneinander beabstandet sind und auf demselben Parallelkreis (10) münden;
- daß der Zusammenbau einen zwischenliegenden Gewindering (30) aufweist, der dazu vorgesehen ist, auf die Basis (28) der Stützkuppenschale aufgedreht (32) zu werden, um die Dehnschlitze (3-8) aufzuweiten, und wobei dessen Innenseite radiale Höcker (34, 35) aufweist; und
- daß die Reibnuß (40) Kerben (43-45) aufweist, die regelmäßig voneinander beabstandet längs Mantellinien angeordnet sind, und die ferner eine Ringnut (46) aufweist, die die Kerben (43-45) schneidet, wobei die Anordnung der Kerben (43-45) und die der Ringnut (46) dazu vorgesehen ist, mit den radialen Höckern (34, 35) zusammenzuwirken, um für die Winkelstellung der Reibnuß (40) in der Stützkuppenschale (1) zu sorgen.

2. Gelenkpfanne für eine Hüftprothese nach Anspruch 1, dadurch gekennzeichnet, daß die Reibnuß (40) in der allgemeinen Form einer Kuppel einstückig ausgebildet ist und im wesentlichen drei aneinanderhängende Abschnitte aufweist, nämlich:
. einen ersten, etwa halbkugelförmigen Abschnitt (41), der dazu vorgesehen ist, sich auf dem Boden der Stützkuppenschale (1) abzustützen,
. einen zylindrischen Abschnitt (42), in den die Kerben (43-45) und die Nut (46) eingeschnitten sind,
. einen über die Stützkuppenschale hinausreichenden Kragen (47), auf dem der zwischenliegende Dehnring (30) ruht, wobei der Kragen (47) einen Sockelvorsprung bildet und bezüglich des Äquators (9) der Stützkuppenschale (1) abgeschrägt ist.

3. Gelenkpfanne für eine Hüftprothese nach Anspruch 1 oder 2, dadurch gekennzeichnet, daß die Innenseite (50) der Reibnuß (40) folgendes in Reihe aufweist:
. einen ersten halbkugelförmigen Abschnitt (51), der dazu vorgesehen ist, den Kugelkopf des Prothesenhalses aufzunehmen,
. ein damit verbundener zylindrischer Abschnitt (52), der dazu vorgesehen ist, ein Ausrenken des Kopfes zu verhindern,
. wobei dieser Abschnitt (52) an seinem Umfang mit einer geneigten Fase (53, 54) versehen ist, deren Neigung invers zur Neigung des Kragens (47) ist, und die dazu vorgesehen ist, ein Neigen des Prothesenhalses (6) bei inneren Drehungen der Hüfte zu gestatten.

4. Gelenkpfanne für eine Hüftprothese nach einem der Ansprüche 1 bis 3, dadurch gekennzeichnet, daß die Stützkuppenschale (1) Öffnungen (16-19) aufweist, die zwischen zwei benachbarten Dehnschlitzen (3-8) ausgespart sind, und die dazu vorgesehen sind, Spreizdübel (20, 21) oder Schrauben mit schwammiger Struktur aufzunehmen.

5. Gelenkpfanne für eine Hüftprothese nach einem der Ansprüche 1 bis 4, dadurch gekennzeichnet, daß die Stützkuppenschale (1) und der zwischenliegende Spreizring (30) aus einer Titanlegierung bestehen, wohingegen die Reibnuß (40) aus Polyethylen hoher Dichte besteht.

## Claims

1. Prosthetic hip cup, of the type comprising:
· a support cup (1), the outer face (2) of which has a plurality of expansion slots (3-8) arranged on generatrices, and which are open on the equator (9), and the inner base (28) of which is threaded;
· a friction core (40), of a general cupola shape, intended on the outer side (41) to be inserted in the support cup (1), and on the hemispherical inner side (50) to receive the head fixed to the end of the prosthetic neck of the prosthesis shaft,
characterized:
- in that the expansion slots (3-8) are symmetrical, uniformly spaced and end on the same parallel (10) ;
- in that the assembly comprises a threaded intermediate ring (30), intended to be screwed (32) on the base (28) of the support cup in order to expand the slots (3-8), and the inner face of which has radial bosses (34, 35);
- and in that the friction core (40) has notches (43-45) arranged on generatrices, uniformly spaced, and an annular groove (46) intersecting the said notches (43-45), the combination of notches (43-45) and annular groove (46) being intended to co-operate with the radial bosses (34, 35) in order to ensure the angular positioning of the friction core (40) in the support cup (1).

2. Prosthetic hip cup according to Claim 1, characterized in that the friction core (40) of general cupola shape is monobloc and comprises essentially three attached parts, respectively:
· a substantially hemispherical first part (41) intended to bear on the base of the support cup (1),
· a cylindrical portion (42) in which the notches (43-45) and the groove (46) are cut,
· a collar (47) on which the intermediate expansion ring (30) rests and which projects beyond the support cup, the said collar (47) forming a seat and being bevelled with respect to the equator (9) of the support cup (1).

3. Prosthetic hip cup according to one of Claims 1 and 2, characterized in that the inner face (50) of the friction core (40) has, in order:
· a first hemispherical portion (51) intended to receive the spherical head of the prosthetic neck,
· connected to a cylindrical portion (52) intended to prevent the dislocation of this head,
· connected in turn to a chamfer (53, 54) inclined counter to the inclination of the collar (47), intended to permit the inclination of the prosthetic neck (60) during the internal rotations of the hip.

4. Prosthetic hip cup according to one of Claims 1 to 3, characterized in that the support cup (1) has orifices (16-19) formed between two contiguous expansion slots (3-8), intended to receive expandable pins (20, 21) or cancellous bone screws.

5. Prosthetic hip cup according to one of Claims 1 to 4, characterized in that the support cup (1) and the intermediate expansion ring (30) are made of titanium alloy, while the friction core (40) is made of high-density polyethylene.
